# EUROPEAN PATENT APPLICATION

(11) **EP 3 398 502 A1**
(43) Date of publication of application: **07.11.2018**
(21) Application number: 17168923.5
(22) Date of filing: 02.05.2017
(51) Int. Cl.: A61B 5/00, A61B 5/02, A61B 1/00, A61B 17/32, A61B 17/3207

(54) **MECHANICAL MATERIAL CHARACTERIZATION IN A PATIENT BODY**

(71) Applicant: Koninklijke Philips N.V., 5656 AE Eindhoven (NL)
(72) Inventor: HAKKENS, Franciscus Johannes Gerardus, 5656 AE Eindhoven (NL); LUCASSEN, Gerhardus Wilhelmus, 5656 AE Eindhoven (NL); HILGERS, Achim, 5656 AE Eindhoven (NL); HENDRIKS, Cornelis Petrus, 5656 AE Eindhoven (NL); VAN DE MOLENGRAAF, Roland Alexander, 5656 AE Eindhoven (NL); VAN DER LINDE, Franciscus Reinier Antonius, 5656 AE Eindhoven (NL); JOHNSON, Mark Thomas, 5656 AE Eindhoven (NL); VAN DEN ENDE, Daan Anton, 5656 AE Eindhoven (NL)
(74) Representative: de Haan, Poul Erik

(57) **Abstract**

The invention relates to an elongated medical device (1), such as a catheter, for insertion into a cavity of a patient body to characterize material arranged in a vicinity of a distal end section of the device (1). The device (1) comprises an actuator formed of a responsive material (34) and disposed at the distal end section of the device (1), which is configured to deform in response to an actuation signal applied to the responsive material (34) to thereby apply a pressure to the material in the vicinity of the distal end region of the device (1). Further, the device (1) comprises a sensor configured to react to a deformation of the actuator and to provide a measurement signal indicative of a resistance of the material against the applied pressure.

## Description

### FIELD OF THE INVENTION

The invention relates to the characterization of material within a patient body. More specifically, the invention is related to an elongated medical device for insertion into a cavity of a patient body to characterize material present therein. Further, the invention is related to a system for characterizing material in a patient body using the elongated medical device.

### BACKGROUND OF THE INVENTION

In many in-vivo treatment procedures, in which medical devices are inserted into body cavities of a patient body to carry out a treatment, it is desirable to determine information about the mechanical characteristic of material in contact with the medical devices, particularly with their tips. Such information maybe used to determine the type of material. Moreover, such information maybe used to adapt the treatment to the specific mechanical properties of the material to be treated.

Examples of such treatment procedures include endovascular catheter-based treatments such as atherectomy and thrombectomy, in which plaque or thrombi is/are removed from blood vessels using a catheter. In order to carry out such procedures, the catheter has to be steered such that its tip is in contact with the material to be removed. For this purpose information about the mechanical properties of material touched by the tip of the catheter may be used, e.g. in order to distinguish whether the tip is contact with the material to be removed, which may be hard calcified material, or with the wall of the blood vessel. Further, the treatment may be adapted to the mechanical properties of the material to be removed, e.g. by adapting a pushing force or - when the catheter drills into material - by adapting the speed of rotation.

Moreover, information about the type of material touched by the catheter tip can be useful in assisting a surgeon in the maneuvering of a catheter, a guidewire or a similar device within the blood vessels towards a treatment position. For instance when crossing a stenosis through the true or subintimal lumen, such information can allow to determine whether the device is currently pushed into the stenosis, against the wall of the blood vessel or through the subintimal lumen. When crossing a stenosis through the subintimal lumen, such information can also be useful for judging whether the tip of the device has passed the stenosis and can be re-entered into the true lumen, for example.

In order to characterize material, ultrasound-based and endoscopic methods may be applied. However, such methods are relatively complex and costly. Moreover, such methods do only allow for recognizing a type of material but do not allow for determining the mechanical properties of the specific material, such as its specific elasticity.

US 2005/0065426 describes a device for characterizing tissue present in a predetermined location of a patient body, which generates mechanical vibrations and measures a frequency response spectrum to characterize the tissue. In this manner, the device allows for determining the mechanical properties of tissue. However, the device is generally used in non-invasive examination procedures so that it is not possible to characterize material in in-vivo treatment procedures. The device can be adapted for use in minimally invasive (e.g. catheter-based) in-vivo procedures. However, the device includes relatively complex components which are generally relative large. Therefore, a large effort is required to miniaturize the device in such a way that it can be used in in-vivo procedures.

### SUMMARY OF THE INVENTION

It is an object of the invention to provide a device which allows for mechanical material characterization in in-vivo procedures in a simpler manner.

In accordance with one aspect, the invention proposes an elongated medical device for insertion into a cavity of a patient body to characterize material arranged in a vicinity of a distal end section of the device. The device comprises an actuator comprising a responsive material and being disposed at the distal end section of the device, the actuator being configured to deform in response to an actuation signal applied to the responsive material to thereby apply a pressure to the material in the vicinity of the distal end region of the device. Moreover, the device comprises a sensor configured to react to a deformation of the actuator and to provide a measurement signal indicative of a resistance of the material against the applied pressure.

Thus, the invention proposes a sensor arrangement that allows for determining a mechanical characteristic of material by applying pressure to the material by means of actuator comprising a responsive material which deforms in response to an actuation signal applied thereto. Such an actuator can easily be integrated into an elongated medical device used in in-vivo procedures and allows for a relatively easy mechanical characterization of material.

The elongated medical device may particularly be a catheter or guidewire. Such elongated medical devices usually provide only a limited space for integrating sensor arrangements. However, due to the aforementioned configuration of the sensor arrangement according to the invention, an easy integration into the space available in such a device is possible.

The responsive material may be an electroactive material, particularly an electroactive polymer. Such materials allow for constructing miniaturized actuators which can be integrated into elongated medical devices having a limit space, such as particularly the aforementioned catheters and guidewires. In case the responsive material is an electroactive material, the actuation signal may correspond to an electrical signal applied to the electroactive material. The actuator comprising the electroactive may be configured to deform in a predetermined manner in response to an application of the electrical signal to the electroactive material.

In one embodiment, the sensor is a deformation sensor configured to provide a measurement signal indicative of an amount of deformation of the actuator. When an actuation signal with a certain value is applied to the actuator, the actual deformation of the actuator can serve as a measure of the resistance of the material against the pressure applied by the actuator. Therefore, it is possible to determine one or more mechanical characteristic(s) of the material, such as the material's elasticity, on the basis of the measured deformation of the actuator. Moreover, the value of the actuation signal may be taken into consideration in the determination of the mechanical characteristic(s).

In a related embodiment, the deformation sensor comprises a strain sensitive foil coupled to the responsive material. The strain sensitive foil may particularly comprise a polyvinylidene fluoride (PVDF) film or a strain gage.

In one embodiment, the actuator is configured to bend in response to the actuation signal applied to the responsive material. In a related embodiment, the elongated medical device further comprises a flexible membrane for sealing the medical device, wherein the actuator is configured to push the flexible membrane against the material to be characterized in response to a bending of the actuator.

In one embodiment the flexible membrane covers a tip of the device. In this embodiment, it is possible to characterize material arranged in front of the tip of the elongated medical device. Thus, it is particularly possible to characterize material into which the elongated device may be pushed while it is steered through a cavity of a patient body, such as a blood vessel.

However, the invention is not limited to his arrangement of the membrane and the sensor arrangement. Likewise, the sensor arrangement may be arranged at one side of the elongated medical device in order to characterize material positioned laterally with respect to the elongated medical device. This is particularly advantageous in the procedure of crossing a stenosis through the subintimal lumen in order to determine a location of a re-entry into the true lumen.

In one embodiment, the elongated medical device further comprises a plunger cooperating with the actuator, the plunger being arranged to be displaced in response to the deformation of the actuator to apply pressure to the material. In a related embodiment, the elongated medical device further comprises a flexible membrane for sealing the medical device, wherein the plunger is configured to push the flexible membrane against the material in response to a bending of the actuator.

It is an advantage of these embodiments that the plunger allows for indenting material on a smaller surface area. As a consequence, the influence of the stiffness of the elongated medical device in the direction of the indention is reduced since the force applied to the elongated medical device due to the pushing of the material is smaller. Thus, this embodiment is particularly suited for elongated medical devices have a lower stiffness in the direction of indention. Moreover, this embodiment is particularly suited for characterizing harder materials.

In a further related embodiment, the deformation sensor is configured to react to a displacement of the plunger and to provide a measurement signal indicative of the displacement of the plunger. In such a way, an especially easy measurement of the deformation is possible. The corresponding sensor for measuring the displacement may be configured as an inductive displacement sensor, for example.

In a further embodiment, the elongated medical device comprises a bendable distal end section, wherein the actuator is arranged to bend the tip portion in response to the electrical signal applied thereto. In this embodiment, it is likewise possible to characterize material arranged laterally to the distal end section of the elongated medical device.

Moreover, one embodiment includes that the actuator cooperates with a cap forming a tip of the device and that the actuator is configured to extend in a longitudinal direction of the device in response to the actuation signal applied to the responsive material to push the cap against the material to be characterized. This embodiment is particularly suited for use in connection with a guidewire having a cap at its distal tip.

Further, the invention proposes a system comprising the elongated medical device and further comprising an evaluation unit configured to determine at least one mechanical property of the material to be characterized based on the actuation signal applied to the responsive material and based on the measurement signal.

In one embodiment of the system, the measurement signal is indicative of an amount of deformation of the actuator and the evaluation unit is configured to determine a parameter characterizing an elasticity of the material based on a ratio between a change of the actuation signal applied to the responsive material and a change of the measurement signal.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily shows an elongated medical device for determining mechanical properties of material within a patient body comprising a sensor arrangement having an actuator to push against material,
Fig. 2 schematically and exemplarily shows a diagram illustrating a dependency between a measurement signal of a deformation sensor and an electrical signal for actuating the actuator,
Fig. 3 schematically and exemplarily shows an EAP layer structure forming the actuator in one embodiment,
Fig. 4A and 4B schematically and exemplarily illustrated a sensor arrangement of the device in one embodiment,
Fig. 5 schematically and exemplarily shows an EAP layer structure forming the actuator in a further embodiment,
Fig. 6 schematically and exemplarily shows a capacitive sensor for measuring a deformation of the EAP layer structure,
Fig. 7 schematically and exemplarily illustrated a sensor arrangement of the device arranged at a side of the elongated medical device,
Fig. 8 schematically and exemplarily illustrated a sensor arrangement of the device in a further embodiment,
Fig. 9A and 9B schematically and exemplarily show inductive sensors for measuring a displacement of a plunger cooperating with the EAP layer structure,
Fig. 10 schematically and exemplarily illustrated a sensor arrangement of the device in a further embodiment, and
Fig. 11 schematically and exemplarily illustrated a sensor arrangement of the device in a further embodiment.

### DETAILED DESCRIPTION OF EMBODIMENTS

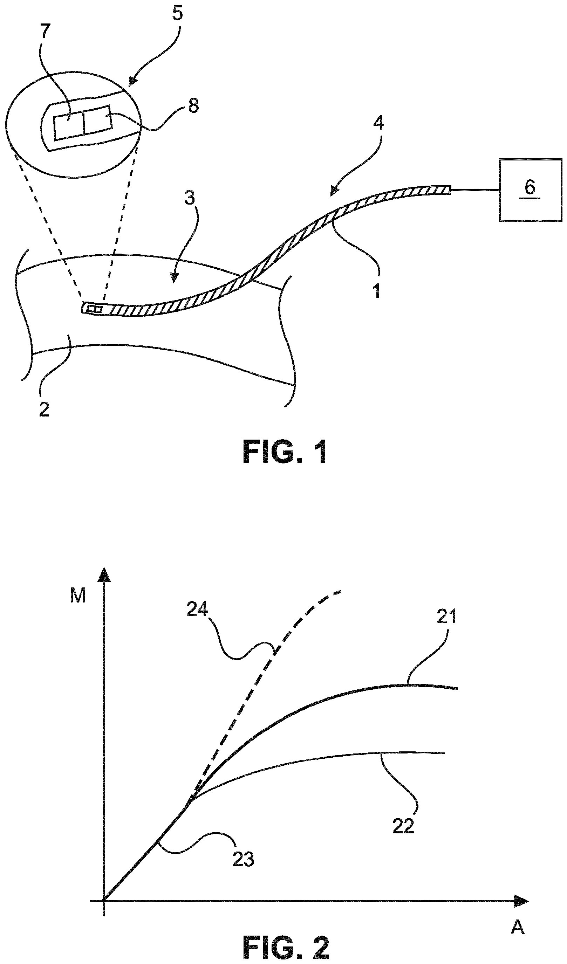

Fig. 1 schematically and exemplarily illustrates components of a system for determining mechanical properties of material touched by a distal portion of an elongated medical device 1. In particular, the system is configured to determine an elasticity parameter of the material, such as an elastic modulus.

The elongated medical device 1 is configured to be inserted into a lumen of patient body 2, such as, for example a blood vessel and/or a lumen of the heart. In specific implementations, the elongated medical device 1 is configured as a catheter or a guidewire, for example. In particular, the elongated medical device 1 may be configured as an ablation catheter, an EP (electrophysiology) catheter, a CTO (chronic total occlusion) crossing catheter, a stent delivery catheter or a guidewire. Likewise, the elongated medical device may be another atherectomy devices, for example.

The elongated medical device 1 has a distal end section 3, which is inserted into the lumen of the patient body 2 in use of the elongated medical device 1. Further, the elongated medical device 1 has a proximal end section 4, which stays out of the patient body 2 also in use of the elongated medical device 1 and which may be operated to maneuver the elongated medical device 1 within the patient body 2. The distal end section 3 comprises a sensing arrangement 5 for sensing one or more mechanical properties of tissue or other material touched by the distal end section 3. The sensing arrangement 5 is electrically coupled to an evaluation unit 6 arranged outside the patient body 2. The coupling may be achieved by means of a wired connection through the elongated medical device 1.

The sensing arrangement 5 comprises an actuator 7 which deforms when an actuation signal is applied thereto and, more specifically, to a material included in the actuator 7 as will be explained further below. Further, the actuator 7 is configured and arranged in such a way that a pressure is applied to material in the vicinity of the distal end section 3 of the elongated medical device 1 when the actuator 7 deforms. In particular, the actuator 7 may be arranged and configured such that material in front of the distal tip of the elongated medical device 1 or arranged laterally to the distal end section 3 of the elongated medical device 1 is pushed against in response to a deformation of the actuator 7. The actuation signal for controlling the actuator 7 may be provided by means of the evaluation unit 6 via a wired connection through the elongated medical device 1.

Further, the sensing arrangement 5 comprises a deformation sensor 8 configured to measure a deformation of the actuator 7. The deformation sensor 8 maybe arranged at the actuator 7 to substantially directly measure a deformation of the actuator 7, or it may indirectly measure a deformation of the actuator 7, e.g. by measuring a displacement of a further component of the sensing arrangement 5 coupled to the actuator 7. The output of the deformation sensor 8 may be an electrical signal, the value of which is indicative of the deformation of the actuator 7. This electrical signal is transmitted to the evaluation unit 6 via a connection through the elongated medical device 1 and evaluated in the evaluation unit in order to determine the one or more mechanical properties of material in the vicinity of the distal end portion 3 of the elongated medical device 1.

The evaluation unit 6 may particular determine the elasticity of the material pushed against due to the deformation of the actuator 7. For this purpose, the evaluation unit 6 may vary the actuation signal and may evaluate the measurement signal of the deformation sensor 8 acquired while varying the actuation signal.

In particular, the evaluation unit 6 may determine the ratio between a change of the actuation signal and a related change of the measurement signal and may determine the elasticity of the material on the basis of this ratio.

In this respect, Fig. 2 schematically and exemplarily shows a diagram in which the measurement signal M is plotted as a function of the actuation signal A for different situations, represented by the curves 21 and 22. The curves 21, 22 are based on the assumptions that higher values of the actuation signal A result in a greater deformation of the actuator 7 and that a greater deformation of the actuator 7 results in higher values of the measurement signal M. Both curves 21, 22 include the common section 23. This section represents the deformation of the actuator 7 before contact is made to material. The further sections of the curves 21, 22 show measurement signals acquired for the further deformation of the actuator 7 when is pushes (directly or indirectly) against softer material (curve 21) or against harder material (curve 22). In addition, the dashed curve 24 illustrates a measurement signal that is acquired in case the actuator 7 further deforms without contact to material.

As can be seen in Fig. 2, both curves 21 and 22 exhibit a greater slope than the dashed curve 24. Moreover, the curve 22 (harder material) has a greater slope than the curve 21 (softer material). Therefore, the evaluation unit 6 may determine the elasticity of the material on the basis of a the ratio ΔM/ΔA between a change ΔM of the measurement signal M and a related change ΔA of the actuator control signal A, which represents the slope of the measurement signal M as a function of the actuation signal A. In this determination, a smaller ratio ΔM/ΔA results in a lower determined elasticity (i.e. harder material) than a larger ratio ΔM/ΔA and vice versa.

In order to perform a quantitative assessment of the elasticity of the material, a calibration may be performed for the elongated medical device 1 and as a result of the calibration a function may be determined which assigns values of the ratio ΔM/ΔA to a material parameter representing the elasticity of the material, such as the elastic modulus. The function may then be stored in the evaluation unit 6 in a suitable format and used for determining the elasticity of material during operation of the elongated medical device 1 by measuring ΔM and ΔA during an actuation of the actuator on the basis of a varying actuation signal.

A measurement of the elasticity of material by means of the sensor arrangement may be initiated manually, e. g. by a physician steering the elongated medical device. For instance, the physician may initiate the measurement when he feels that the elongated medical device 1 is pushed against material, such as, for example, a stenosis. In addition or as an alternative, images of the inner of a patient body may be acquired using a suitable imaging modality, such as, for example, computed tomography imaging or ultrasound imaging, in order to assist the physician in steering the elongated medical device and the physician may initiate a measurement when he determines on the basis of the images that the elongated medical device 1 is in contact with material in the region of the sensor arrangement 5.

In alternative embodiments, a measurement may also be initiated automatically. In such embodiments, the elongated medical device 1 may comprise a further sensor reacting to an applied pressure in the vicinity of the sensor arrangement 5 and a measurement may be initiated in case it is determined on the basis of the measurement signals of the further sensor that the elongated medical device 1 is in contact with material.

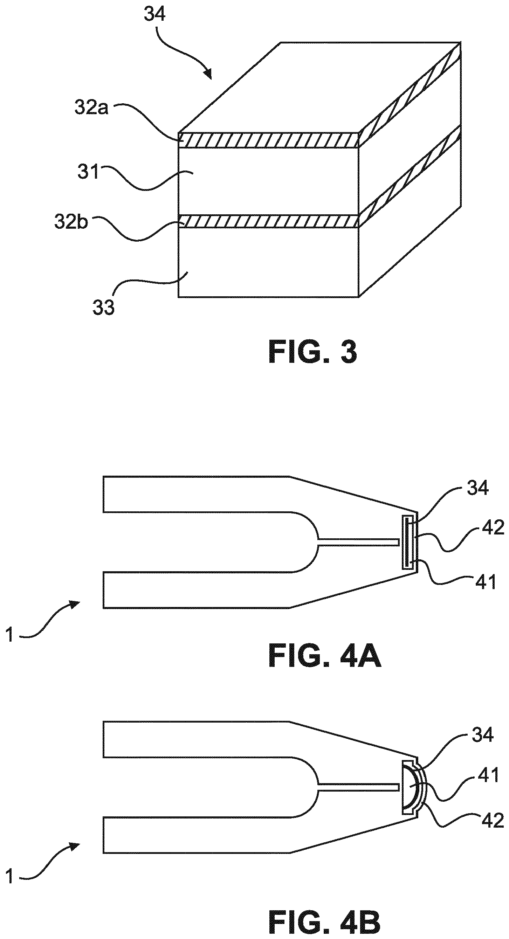

The actuator 7 comprises an electroactive material, which may be an electroactive polymer (EAP) 31, for example. The EAP 31 may be a field-driven material, in which actuation is forced by electrostatic forces, such as, for example, a dielectric EAP, a polyvinylidene fluoride (PVDF)-based relaxor polymer or a liquid crystal elastomer (LCE). Likewise, the EAP 31 may be an ionic-driven material, in which the actuation is caused by the displacement of ions in the polymer, such as, for example, a conjugated polymer, a carbon nanotube (CNT)-polymer composite or an ionic polymer-metal composite (IPMC). In order to actuate the EAP 31, it may be provided between two flexible electrode layers 32a,b as schematically and exemplarily illustrated in Fig. 3a, and the actuator control signal may be applied to these electrode layers 32a, 32b. Further, the EAP 31 and the electrode layers 32a, 32b may be arranged on a passive support layer 33. The EAP 31 and the electrode layers 32a, 32b together form an EAP layer structure 34. In addition, the EAP layer structure 34 may optionally comprise a layer forming part of the deformation sensor 8 as will be further explained herein below.

In first embodiments, the EAP 31 is included in an opening arranged at the distal end portion 3 of the elongated medical device 1. Further, the EAP layer stack is configured to bend towards the distal end of the elongated medical device 1 in response to an applied electrical signal. Hereby, pressure is applied to material in front of the distal tip of the elongated medical device 1.

One related implementation is schematically and exemplarily illustrated in Figs. 3A and 3B. In this implementation, the EAP layer stack is arranged in an opening that is closed by a flexible membrane 42 towards the distal tip of the elongated medical device 1. When no electrical signal is applied to the EAP 31, the EAP layer structure 34 is substantially flat as shown in Fig. 4A. In the opening, the EAP layer structure 34 may be positioned such that it is in contact with the membrane 42 when not electrical signal is applied to the EAP 31. However, there may likewise be a small gap between the layer structure 34 and the membrane 42 in this situation. When an actuation signal is applied to the EAP 31, the EAP layer structure 34 bends towards the distal end of the elongated medical device 1, i.e. towards the flexible membrane 42. Hereby, the EAP layer structure 34 pushes against the membrane 42 so that the membrane 42 bends in the same direction and pushes against material in front of the distal tip of the elongated medical device 1.

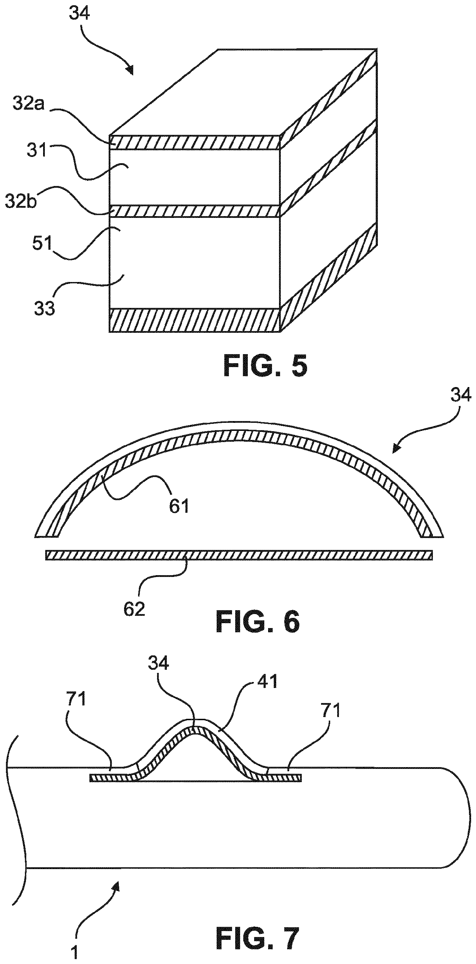

In order to determine the elasticity of the material as explained above, the deformation of the EAP layer structure 34 is determined using the deformation sensor 8. In the aforementioned implementation, the deformation sensor 8 may comprise a flexible strain sensitive foil 51, which may be included as a further layer into the EAP layer structure 34. The strain sensitive foil 51 may be arranged below the support layer 33 as schematically and exemplarily illustrated in Fig. 5. At this position, the greatest compressive stress can be measured within the layer structure 34 when the layer structure 34 bends (in upward direction). However, the strain sensitive foil 51 may likewise be arranged at another position within the EAP layer structure 34. In particular, the strain sensitive foil 51 may be arranged on top of the upper electrode layer 34. At this position, the greatest strain can be measured when the layer structure 34 bends. Moreover, the strain sensitive foil 51 maybe configured as a continuous layer within the EAP layer structure 34 or it maybe configured as a segmented foil that is only included in certain sections of the plane of the EAP layer structure 34.

The strain sensitive foil 51 may be configured to provide an electrical signal in response to a strain applied thereto due to the bending of the EAP layer structure 34 and the electrical signal may be collected using electric contacts coupled to the strain sensitive foil 51 and forwarded to the evaluation unit 6. In this configuration, the strain sensitive foil 51 may include a PVDF film, for example. As an alternative, the strain sensitive foil 51 may be configured such that its electrical resistance changes in response to an applied strain. In this case, an electrical current may be supplied be evaluation unit 6, which flows through the strain sensitive foil 51, and changes of the voltage drop across the strain sensitive foil 51 due to a change of its electrical resistance are measured in the evaluation unit 6. In this configuration, the strain sensitive foil may include a strain gage, for example.

In further embodiments, another type of deformation sensor 8 is used, which is not configured as a strain sensitive foil 51. For instance, the deformation sensor 8 may be configured as a capacitive sensor arrangement. Such a sensor arrangement is schematically and exemplarily illustrated in Fig. 6. It may comprise one flexible electrode layer 61, which is part of the EAP layer structure 34 and forms the lowermost layer thereof (the other layers are not depicted individually in Fig. 6). Moreover, it may comprise a fixed and rigid electrode 62 facing the electrode layer 61. The two opposing electrodes 61, 62 are connected to the evaluation unit 6. Further, a ductile dielectric material may be disposed between the electrodes 61 and 62, which allows for pulling the electrodes 61 and 62 apart.

In this type of deformation sensor 8, the two opposing electrodes 61 and 62 form a capacitor. When the EAP layer structure 34 bends in response to an electrical signal applied thereto, the distance between the electrodes 61 and 62 changes and, thus, the capacitance of the capacitor changes. This change is measured in the evaluation unit 6 in order to determine the deformation of the EAP layer structure 34.

In a variant of the aforementioned embodiments, the sensor arrangement 5 including the EAP layer structure 34 is arranged at one side of the distal section 3 of the elongated medical device 1 rather than at the tip thereof. Hereby, it is possible to examine material arranged laterally to the distal section 3 of the elongated medical device 1. This may particularly be advantageous in a procedure for crossing a stenosis through the subintimal lumen. In this procedure, the elasticity of material arranged laterally to the distal section 3 of the elongated medical device 1 may particularly be analyzed in order to determine a location for a re-entry into the true lumen.

In order characterize laterally arranged material, the sensor arrangement 5 may be operated to determine the elasticity of this material when the opposite side of the elongated medical device 1 is likewise in contact with material. For instance, this is typically the case when the elongated medical device 1 is used to cross a CTO. When the opposite side of the elongated medical device 1 is likewise in contact with material, the elongated medical 1 is hold in place during the measurement carried out by means of the sensing arrangement 5. Further, since the surface area of the elongated medical device 1 contacting the material at the opposite side is considerable larger than the area in which material is pushed against by means of the sensor arrangement 5, the stiffness of the material at the opposite side only has a small influence on the measurement carried out by means of the sensor arrangement 5. Thus, a relatively accurate determination of the elasticity of the material in front of the sensor arrangement 5 is usually possible.

In order to determine whether the opposite side of the elongated medical device 1 is in contact with material, there may be a further sensor arrangement at the opposite side of the elongated medical 1. This sensor arrangement may be configured in the same manner as the sensor arrangement described above. Using the measurement signals of this sensor arrangement, it may be determined that the elongated medical 1 is contact with material at the side of this sensor arrangement in case the sensed stiffness is greater than a predetermined threshold.

In case the sensor arrangement 5 is arranged at one side of the elongated medical device 1 to characterize laterally arranged material, it may generally be configured in the same manner as explained above. Thus, the included EAP layer structure 34 may be mounted within an opening 41, which is sealed by means of a flexible membrane 42 towards the respective side of the elongated medical device 1, and the EAP layer structure 34 may be actuated to push against the flexible membrane 42 and, thus, to push against material to be characterized in front of the flexible membrane 42. Moreover, the opening 41 and the sensor arrangement 5 may be larger in size compared with the sensor arrangement arranged at the distal tip of the elongated medical device 1. In particular, the EAP layer structure 34 may have a larger extension in a direction perpendicular to its bending direction, i.e. in the direction of the longitudinal extension of the elongated medical device 1. As schematically and exemplarily depicted in Fig. 7, the EAP layer structure 34 may particularly be arranged in the opening 41 such that its edges are hold in fixtures 71. In this configuration, the EAP layer structure 34 may completely expand in response to an application of an electrical signal but - since the edges are fixated - only the middle portion may bend. In this way, a higher pressure can be applied to the material pushed against by means of the EAP layer structure 34.

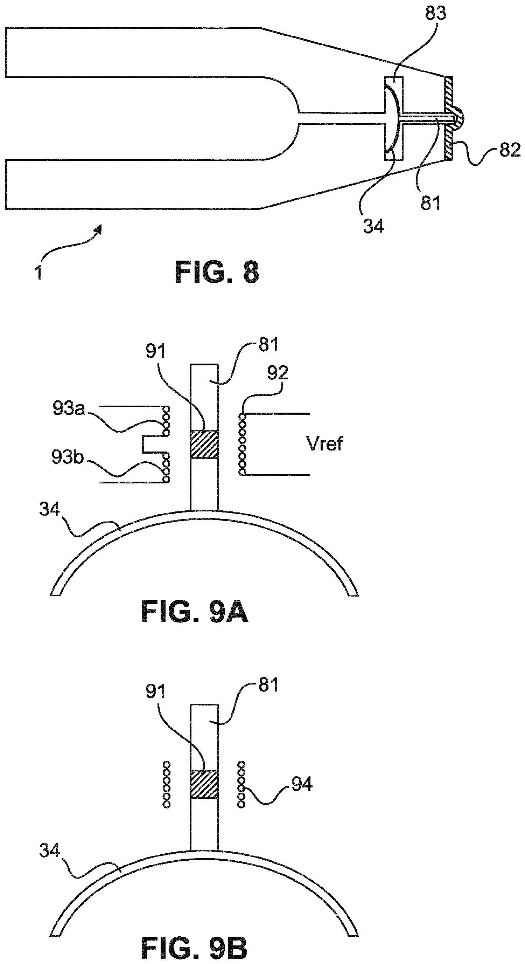

A further embodiment of the sensing arrangement 5 differs from the aforementioned embodiments particularly in that the EAP layer structure 34 does not directly push against a membrane which is in contact with the material to be examined. Rather, the EAP layer structure 34 drives a plunger 81 which pushes against the material via a membrane 82. Such an embodiment is schematically and exemplarily illustrated in Fig. 8.

As shown in Fig. 8, the EAP layer 34 structure is arranged in an opening 83 having a certain distance from the distal tip of the elongated medical device 1. The EAP layer structure maybe configured as described above. Further, a plunger 81, which is oriented along the longitudinal direction of the elongated medical device 1, extends into the opening 83 and contacts the EAP layer structure 34 with one end. The other end of the plunger 81 extends to the tip of the elongated medical device 1 through a bore, which is covered by a flexible membrane 82 at the tip of the elongated medical device 1. Within the bore, the plunger 81 can move back and forth in the direction of the longitudinal extension of the elongated medical device 1.

In this arrangement, the plunger 81 is displaced in the direction towards the tip of the elongated medical instrument 1, when the EAP layer structure 34 bends in response to an electrical signal applied thereto. As a consequence, the plunger 81 pushes against material to be examined which is arranged in front of the tip of the elongated medical device 1. The amount of displacement of the plunger 81 and, thus, the amount of deformation of the EAP layer structure 34, as a function of the voltage applied to the EAP layer structure 34 varies in dependence of the elasticity of the material to be examined. Therefore, it is possible to assess the material's elasticity in the same way as described above on the basis of a measurement of a deformation of the EAP layer structure 34 or a parameter indicative thereof using the deformation sensor 8.

For this purpose, the deformation sensor 8 may directly measure the deformation of the EAP layer structure 34 and may be configured in a manner described above. In particular, the deformation sensor 8 may comprise a strain sensitive foil 51. In this case, the strain sensitive foil 51 may be segmented and may spare a region in which the EAP layer structure 34 contacts the plunger 81. Hereby, it can be prevented that the force applied by the plunger 81 disturbs the measurements.

As an alternative, the deformation sensor 8 may include a sensor arrangement configured to measure the displacement of the plunger 81. In this alternative, the deformation sensor 8 maybe configured as an inductive sensor. In this variant, the plunger 81 may comprise one marker section 91 having a different permeability than the other sections. Further, a coil arrangement may be provided in the vicinity of the plunger 81 which is connected to the evaluation unit 6 and which is arranged such that the marker section 91 moves through the coil arrangement when the plunger 81 is displaced. Thus, an electrical signal is induced in the coil arrangement which is indicative of the displacement of the plunger 81. Using this signal, the evaluation unit 6 can determine that elasticity of the material to be examined as explained above.

In one related implementation schematically and exemplarily shown in Fig. 9A, the inductive sensor is configured as a linear variable transformer. It includes a primary coil 92 and co-axial secondary coils 93a,b surrounding the plunger 91. The secondary coils 93a,b are connected in series and are preferably configured identically. To the primary coil 92, an alternating reference voltage Vref is applied. This reference voltage Vref may be supplied by the evaluation unit 6. The resulting alternating current flowing through the primary coil 92 induces a voltage in each of the two serially connected secondary coils 93a and 93b, which is measured in the evaluation unit 6. The coils are arranged such that the marker section 91 of the plunger 81 moves within the coils when the plunger 81 is displaced, so that the voltage induced in the secondary coils 93a,b changes in response to the displacement of the plunger 81, and the evaluation unit 6 may sense this change in order to determine the displacement of the plunger 81.

More specifically, equal voltage levels are induced when the marker section 91 is at arranged at the middle position of the secondary coils 93a,b, i.e. when it extends into both secondary coils 93a,b over the same length. When the marker section 91 moves from the middle position, the voltages induced into the secondary coils 93a,b change differently. Thus, the evaluation unit 6 can determine the position of the plunger 81 on the basis of the voltage signals provided by the secondary coils 93a, 93b.

In a further, simpler configuration, which is schematically and exemplarily illustrated in Fig. 9B, the coil arrangement comprises a single coil 94 which surrounds the plunger 81 and is arranged such that the plunger 81 and particularly its marker section 91 move within the coil 94 when the plunger 81 is displaced. Hereby, the marker section 91 changes the inductivity of the coil 94 when it moves through the coil 94, where the value of the inductivity at a certain position of the plunger 81 particularly depends on the length of the part of the marker section 91 which is positioned within the coil 94 in this position of the plunger 81. In order to determine the change of the coil's inductivity, the evaluation unit 6 may drive an alternating current flowing through the coil 94 and may evaluate the voltage drop across the coil 94 and the value of the current.

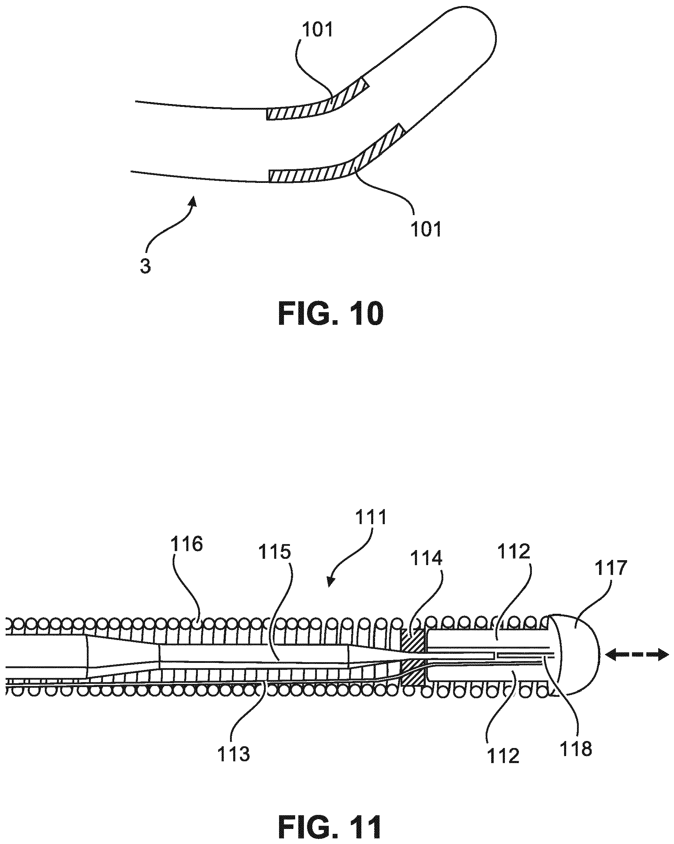

Furthermore, one embodiment of the elongated medical device 1 comprises that the distal end section 3 of the elongated medical device 1 can be bended as schematically and exemplarily shown in Fig. 10. For this purpose, the distal end section 3 maybe formed of a sufficiently flexible material at least in a region in which the distal end section 3 is bent. Further, the tip section on the distal side of the bending region may be formed of a sufficiently stiff material so that the tip can be pushed against material as explained below without the tip being deformed too much. In order to bend the distal end section 3, one or more bending actuator(s) 101 is/are provided within the bending section of the distal end section 3. Each bending actuator may comprise an EAP layer structure 34 which bends in response to an electrical signal applied thereto and which may be configured in a manner described above. Further, each bending actuator is provided with a deformation sensor 8 (not shown in Fig. 9), which may be configured as a strain sensitive foil 51 included in the EAP layer structure 34 as explained above.

When the distal end section 3 of the elongated medical device 1 is bended by means of the bending actuator 101 in response to the electrical signal applied to the EAP layer structure 34 included therein, the tip of the elongated medical device 1 is pushed against tissue or other material in the vicinity of the tip. The elasticity of the material may by determined by the evaluation unit 6 based on the electrical signal and the measurement signal of the deformation sensor 8 as explained above.

Also in this embodiment, the measurement is preferably carried out when the both sides of the elongated medical device 1 are in contact with material so that the opposite side of the elongated medical device 1 is supported by material when the tip is pushed against material at one side of the elongated medical device 1. If this is the case, the elongated medical device 1 stays in position during the measurement and the elasticity of the material in contact with the tip of the elongated medical device 1 can be measured. The elasticity or stiffness of the material contacted on the opposite side of the elongated medical device 1 only has a small influence on the result of the measurement since the contact surface on this side is (much) larger than the contact surface of the tip.

Further embodiments differ from the embodiments explained above in that the actuator 7 does not bend but rather expands in the longitudinal direction of the elongated medical device 1 in response to the actuation signal applied to the actuator 7. One related implementation, in which the elongated medical device 1 is configured as a guidewire 111, is schematically and exemplarily illustrated in Fig. 11. In this implementation, the actuator 7 comprises an EAP 112 configured to expand in the longitudinal direction of the guidewire 111 when an electrical signal is applied thereto, which may be provided by the evaluation unit 6 through connection wires 113. At its proximal side, the EAP 112 is supported by a ring 114 which is fixedly mounted on the core 115 of the guidewire 101. At its distal side, the EAP 112 is in contact with a cap 117 forming the tip of the guidewire 111. The cap 117 may be connected to the coil 116 of the guidewire 111. However, the cap 115 is not fixedly connected to the core 115 of the guidewire, so that it can be displaced in the longitudinal direction of the guidewire 111 relative to the core 115.

When the EAP 112 expands in response to the electrical signal applied thereto, the EAP 112 pushes the cap 117 against material arranged in front of the tip of the guidewire 111. The elasticity of this material is again determined by the evaluation unit 6 on the basis of the electrical signal and a parameter indicative of an amount of deformation (expansion) of the EAP 112 as explained above. The amount of deformation is determined using a deformation sensor 8 which may comprise a strain sensitive foil coupled to the EAP 112 as explained above. As an alternative, the deformation sensor 8 may comprise a sensor arrangement configured for measuring the displacement of the cap 117. This could be done by measuring the displacement of a rod 118 coupled to the cap and extending into the proximal direction of the guidewire 111 towards its core 115. The displacement of this rod 118 may be measured using a displacement sensor 8 of the type described above in connection with measuring the displacement of the plunger 81.

Using an elongated medical device 1 configured in accordance with one of the embodiments described above, it is possible to determine the elasticity of material arranged in the vicinity of the distal end section 3 of the elongated medical device. However, the invention is not limited to these embodiments. So, as already said above, the actuator 7 may comprise another electroactive material, such as for example, a piezo ceramic. In further embodiments, the actuator 7 may comprise another responsive material, which is not actuated using an electrical signal but using another form of actuation. For instance, the actuator may comprise a shape memory alloy which deforms in response to a temperature change. This material may shrink at higher or lower temperatures and on the basis of this characteristic a bending actuator maybe formed from this material in combination with other materials (which not bend in response to temperature changes). In order to actuate the material, a cooling or heating element may be integrated into the sensor arrangement which may be operated on the basis of a suitable actuation signal to cool or heat the shape alloy material so that the actuator 7 bends similar as in the embodiments explained above.

Further, the configuration of the deformation sensor 8 may differ from the configurations exemplarily described in connection with the aforementioned embodiments. Moreover, the sensor arrangement 5 may comprise a sensor for measuring a force applied to the responsive material instead of or in addition to a deformation sensor 8 configured in a manner described above. Using the force sensor, the elasticity of material pushed against by means of the sensing arrangement 5 may be determined on the basis of the measured force and on the basis of the actuation signal applied to the responsive material. In this determination, a higher measured force at a certain value of the actuation signal is usually indicative of a higher stiffness of the material. In order to carry out a corresponding quantitative determination of the stiffness or elasticity, a calibration may again be carried out in advance to determine a function for associating stiffness or elasticity values to values of the actuation signal and the measured force signal and this function may be stored in the evaluation unit 6 for use during the measurements.

The force sensor may include a piezoceramic material and/or may be configured as a capacitive sensor. Likewise, in case the responsive material comprises an EAP as in the embodiments described above, the force applied to the sensor arrangement 5 may be measured using the EAP itself. For instance, this may be done in a way described in WO 2017/037117 on the basis of a measurement of an impedance of the EAP material.

In another variation of the embodiments explained in detail above, the actuator 7 of the sensor arrangement 5 may be actuated cyclically with different frequencies during a measurement. Then, the response of the material pushed against at the different frequencies may be measured. The frequency response of the material varies depended upon the mechanical properties of the material, particularly depending upon the material's elasticity. Therefore, the elasticity or other mechanical characteristics of the material may be determined on the basis of the frequency response.

Furthermore, it is also possible that the evaluation unit 6 is not arranged external to the elongated medical 1 but integrated therein. In such an implementation, the evaluation unit 6 may particularly be configured as an ASIC (application-specific integrated circuit) which may be arranged within the elongated medical device 1 and which may comprise functionality for controlling the actuator 7 and for determining the elasticity of the material to be examined on the basis of the measurement signal of the deformation sensor 8.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. An elongated medical device (1) for insertion into a cavity of a patient body to characterize material arranged in a vicinity of a distal end section (3) of the device (1),
the device (1) comprising an actuator (7, 101) comprising a responsive material (31, 112) and being disposed at the distal end section (3) of the device (1),
the actuator (7, 101) being configured to deform in response to an actuation signal applied to the responsive material (31, 112) to thereby apply a pressure to the material in the vicinity of the distal end region (3) of the device (1), and
the device (1) comprising a sensor (8) configured to react to a deformation of the actuator (7, 101) and to provide a measurement signal indicative of a resistance of the material against the applied pressure.

2. The elongated medical device (1) as defined in claim 1, wherein the responsive material (31, 112) is an electroactive material, particularly an electroactive polymer.

3. The elongated medical device (1) as defined in claim 1, wherein the sensor (8) is a deformation sensor configured to provide a measurement signal indicative of an amount of deformation of the actuator (7, 101).

4. The elongated medical device (1) as defined in claim 3, wherein the deformation sensor (8) comprises a strain sensitive foil (51) coupled to the responsive material (31).

5. The elongated medical device (1) as defined in claim 4, wherein the strain sensitive foil (51) comprises a polyvinylidene fluoride film or a strain gage.

6. The elongated medical device (1) as defined in claim 1, wherein the actuator (7, 101) is configured to bend in response to the actuation signal applied to the responsive material (31).

7. The elongated medical device (1) as defined in claim 6, further comprising a flexible membrane (42) for sealing the medical device (1), wherein the actuator (7) is configured to push the flexible membrane (42) against the material to be **characterized in** response to a bending of the actuator (7).

8. The elongated medical device (1) as defined in claim 7, wherein the flexible membrane (42) covers a tip of the device (1).

9. The elongated medical device (1) as defined in claim 1, further comprising a plunger (81) cooperating with the actuator (7), the plunger (81) being arranged to be displaced in response to the deformation of the actuator (7) to apply pressure to the material.

10. The elongated medical device (1) as defined in claim 9, further comprising a flexible membrane (82) for sealing the medical device (1), wherein the plunger (81) is configured to push the flexible membrane (82) against the material in response to a bending of the actuator (7).

11. The elongated medical device (1) as defined in claim 9, wherein the deformation sensor (8) is configured to react to a displacement of the plunger (81) and to provide a measurement signal indicative of the displacement of the plunger (81).

12. The elongated medical device (1) as defined in claim 1, comprising a bendable distal end section (3), wherein the actuator (101) is arranged to bend the distal end section (3) in response to the actuation signal applied to the responsive material (31).

13. The elongated medical device (1) as defined in claim 1, wherein the actuator (7) cooperates with a cap (117) forming a tip of the device and wherein the actuator (7) is configured to extend in a longitudinal direction of the device (1) in response to the actuation signal applied to the responsive material (112) to push the cap (117) against the material to be characterized.

14. A system comprising an elongated medical device (1) as defined in claim 1 and further comprising an evaluation unit (6) configured to determine at least one mechanical property of the material to be characterized based on the actuation signal applied to the responsive material (31, 112) and based on the measurement signal of the sensor (8).

15. The system as defined in claim 14, wherein the measurement signal is indicative of an amount of deformation of the actuator (7, 101) and wherein the evaluation unit (6) is configured to determine a parameter characterizing an elasticity of the material to be characterized based on a ratio between a change of the actuation signal applied to the responsive material (31, 112) and a change of the measurement signal.
